(19) Europäisches Patentamt
European Patent Office
Office européen des brevets

(11) Publication number: **0 093 511**
**B1**

# (12) EUROPEAN PATENT SPECIFICATION

(45) Date of publication of patent specification: **24.09.86**

(51) Int. Cl.$^4$: **C 07 C 61/04,** C 12 P 41/00

(21) Application number: **83301925.0**

(22) Date of filing: **06.04.83**

## (54) Method for producing and optically active 2,2-dimethylcyclopropanecarboxylic acid.

(30) Priority: **12.04.82 JP 61237/82**
**10.01.83 JP 2600/83**
**17.01.83 JP 6348/83**
**09.02.83 JP 20982/83**

(43) Date of publication of application:
**09.11.83 Bulletin 83/45**

(45) Publication of the grant of the patent:
**24.09.86 Bulletin 86/39**

(84) Designated Contracting States:
**CH DE FR GB IT LI**

(56) References cited:
**EP-A-0 034 428**
**CH-A- 545 262**

**Patent Abstracts of Japan, Vol. 6, no. 231, 17 November 1982**

**Patent Abstracts of Japan, Vol. 4, no. 144, 11 October 1980, page 51C27**

(73) Proprietor: **SUMITOMO CHEMICAL COMPANY, LIMITED**
**15 Kitahama 5-chome Higashi-ku**
**Osaka-shi Osaka 541 (JP)**

(72) Inventor: **Minai, Masayoshi**
**1606-5, Harimadacho**
**Moriyama-shi (JP)**
Inventor: **Katsura, Tadashi**
**9, Yamadahigashi-2-chome**
**Suita-shi (JP)**
Inventor: **Hamada, Kazuhiko**
**2-1-248, Kuwatacho**
**Ibaraki-shi (JP)**
Inventor: **Suzukamo, Gohfu**
**2-1-216, Kuwatacho**
**Ibaraki-shi (JP)**

(74) Representative: **Harrison, David Christopher et al**
**MEWBURN ELLIS & CO 2/3 Cursitor Street**
**London EC4A 1BQ (GB)**

**EP 0 093 511 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a method for obtaining an optically active isomer of 2,2-dimethylcyclopropanecarboxylic acid which is useful as an intermediate for dehydropeptidase I inhibitors, medicines and chemicals for use in agriculture, using a resolving reagent.

There are many cyclopropanecarboxylic acid derivatives which have optically active forms, and very many resolving agents have been proposed for resolving racemic mixtures of such acids. For example, α-phenyl-β-(p-tolyl)ethylamine has been used in dl-form to resolve a chrysanthemumic acid containing an excess of a desired optically active form (JP—A—57—13447), in d-form to resolve the racemic trans-isomer of chrysanthemumic acid (CH—A—545262) and the racemic cis, trans 2,2-dimethyl-3-n-butoxycyclopropanecarboxylic acid (JP—A—55—92345) and in d- and l-form to resolve the racemic cis-isomer of 2,2-dimethyl-3-(2,2-dichlorovinyl) cyclopropanecarboxylic acid into its respective d- or l-form (EP—A—0034428).

The conventionally well-known method for obtaining an optically active form of 2,2-dimethylcyclopropanecarboxylic acid is the optical resolution of dl-2,2-dimethylcyclopropanecarboxylic acid, and well-known resolution methods are (1) resolution with quinine (JP—A—51023/1980) and (2) resolution with d- or l-α-phenethylamine (GB—A—1,260,847).

The method (1), however, has problems associated with it, namely that quinine, used as resolving agent, is very expensive and is not available in stable form. Furthermore, the method gives a low yield. The method (2) carries with it the problem that the resultant optically active 2,2-dimethylcyclopropanecarboxylic acid is of such a low optical purity that the optical rotation of its d-form is +65° and that of its l-form is −72°. Neither of these methods, therefore, is suitable as an advantageous industrial process for obtaining an optically active 2,2-dimethylcyclopropanecarboxylic acid of high optical purity. This is especially so because the carboxylic acid is required to have an extremely high optical purity for use as an intermediate for medicines, as a chemical for use in agriculture and as a reagent for measuring optical purity. Hence, conventional methods are, we find, never satisfactory.

Bearing in mind the above, we extensively studied methods of producing an optically active 2,2-dimethylcyclopropanecarboxylic acid of high optical purity in good yields and by a process which can be applied advantageously in industry.

The present invention provides a method for recovering, from 2,2-dimethylcyclopropanecarboxylic acid, an optically active form thereof which method comprises reacting an optically active diphenylethylamine represented by the formula (I),

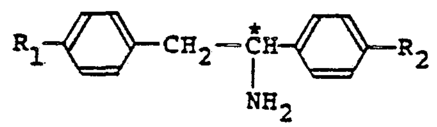

(I)

wherein $R_1$ and $R_2$ are a hydrogen atom or a lower alkyl group, with the 2,2-dimethylcyclopropanecarboxylic acid in a solvent to form a diastereomer salt with the said amine, allowing the salt to deposit, separating the said diastereomer salt from the solvent to thereby separate the optically active forms of the said acid from one another and recovering the desired said optically active form from one of the said salt and the said solvent to achieve the optical purification of said acid.

The present invention will be illustrated in detail hereinafter.

2,2-Dimethylcyclopropanecarboxylic acid used as a starting material may be in its racemic form or the acid may contain the d- or l-form in excess. Such an acid can easily be synthesized, for example, by the method described in J.O.C., *40*, 456 (1975) and Japanese Patent Publication No. 46835/1978.

The optically active diphenylethylamine represented by the formula (1), also used as a starting material, may be, for example, d- or l-α-phenyl-β-(p-tolyl) ethylamine, d- or l-α-(p-tolyl)-β-phenylethylamine or d- or l-α-phenyl-β-phenylethylamine.

The optical purification treatment of 2,2-dimethylcyclopropanecarboxylic acid is carried out by reacting the carboxylic acid with an optically active diphenylethylamine represented by the foregoing formula (I) in a solvent to form a diastereomer salt, and slowly cooling the reaction mixture to deposit one of the optically active diastereomer salts which is then separated and decomposed.

The molar amount of the optically active diphenylethylamine used in formation of the diastereomer salt, is generally from 0.3 to 1.2, preferably 0.5 to 1.05 times the molar amount of the 2,2-dimethylcyclopropanecarboxylic acid.

The solvent used in the above reaction may be, for example, an alcohol such as methanol, ethanol, isopropanol, or n-propanol, a ketone such as acetone or methyl ethyl ketone, an ester such as ethyl acetate or isopropyl acetate, an aromatic hydrocarbon such as toluene or benzene, a halogenated hydrocarbon such as chloroform or dichloromethane, an aliphatic hydrocarbon such as hexane or heptane, an ether such as ethyl ether or tetrahydrofuran, and mixtures thereof. Particularly, however, water-soluble solvents (e.g. methanol, ethanol, isopropanol, n-propanol or acetone), mixtures of these solvents with water and aromatic hydrocarbons are preferably used.

The amount of solvent used is properly determined according to various conditions such as the kind

and optical purity of 2,2-dimethylcyclopropanecarboxylic acid used as a starting material and the kinds of diphenylethylamines and solvents. Generally, however, the amount is in a range of 5 to 100 times by weight based on the weight of 2,2-dimethylcyclopropanecarboxylic acid.

The reaction temperature may optionally be selected within a range of from −20°C to the boiling point of the solvent used, but preferably, it is not less than a temperature at which the formed diastereomer salt is deposited.

After the optionally active diastereomer salts have been formed by the reaction described above, the reaction mixture is slowly cooled to deposit one of the optically active diastereomer salts. The crystals obtained may be purified, if necessary, by repeating recrystallization.

The crystals thus obtained are then filtered off, dried if necessary and decomposed with an acid or alkali. When an acid is used for the decomposition, hydrochloric acid, sulfuric acid, phosphoric acid or acetic acid may be used, and the released optically active 2,2-dimethylcyclopropanecarboxylic acid is extraction-isolated with an organic solvent such as toluene, benzene, chloroform, ethyl acetate or ethyl ether.

When an alkali is used for the decomposition, sodium hydroxide or potassium hydroxide may be used, and by extracting the decomposition solution with an organic solvent, the optically active diphenylethylamine used as a resolving reagent moves to the organic solvent layer, while the optically active carboxylic acid stays as a salt in the aqueous layer. On neutralizing this aqueous layer with addition of an acid and extracting with an organic solvent, the optically active 2,2-dimethylcyclopropanecarboxylic acid is extracted into the organic layer.

In this case, the same acid and organic solvent as described above are used for neutralization and extraction, respectively.

After removing from the filtrate the crystals of one optically active diastereomer salt by filtration as described above, the other diastereomer salt containing an excess of 2,2-dimethylcyclopropanecarboxylic acid having an opposite optical activity, is recovered from the filtrate by removing the solvent therefrom. The acid can be isolated by recrystallizing the diastereomer salt and applying the same decomposition and after-treatment as above, or re-dissolving the diastereomer salt in the foregoing solvent, deposition-separating the salt by adding a seed crystal to the resulting solution and then applying the same decomposition and after-treatment as above to the crystal obtained.

In this way, d- or l-2,2-dimethylcyclopropanecarboxylic acid is obtained in good efficiency, and the optically active phenylethylamine used also can be recovered.

Such optical purification will be illustrated hereinafter with reference to specific compounds.

When the carboxylic acid is a racemic 2,2-dimethylcyclopropanecarboxylic acid and the optically active diphenylethylamine represented by the formula (I) is d-α-phenyl-β-(p-tolyl)ethylamine, a diastereomer salt obtained by deposition is that of l-2,2-dimethylcyclopropanecarboxylic acid with d-α-phenyl-β-(p-tolyl)-ethylamine, the diastereomer salt of d-2,2-dimethylcyclopropanecarboxylic acid with d-α-phenyl-β-(p-tolyl)ethylamine being present in the filtrate. This means, therefore, that the l-2,2-dimethylcyclopropanecarboxylic acid is obtained in crystal form by applying the aforementioned operation, while the d-2,2-dimethylcyclopropanecarboxylic acid is obtained by further purification of the filtrate part. When the foregoing d-form α-phenyl-β-(p-tolyl)ethylamine is replaced by the l-form, an opposite result to the foregoing is obtained, that is, d-2,2-dimethylcyclopropanecarboxylic acid is obtained from the crystal part and l-2,2-dimethylcyclopropanecarboxylic acid is obtained by purifying the filtrate part.

But, when d-α-phenyl-β-(p-tolyl)ethylamine is replaced by d-α-phenyl-β-phenylethylamine, a diastereomer salt obtained from the crystal part is that of d-2,2-dimethylcyclopropanecarboxylic acid with d-α-phenyl-β-phenylethylamine, the diastereomer salt of l-2,2-dimethylcyclopropanecarboxylic acid with d-α-phenyl-β-phenylethylamine being present in the filtrate part. Consequently, d-2,2-dimethylcyclopropanecarboxylic acid is obtained from the crystal part by applying the foregoing operation and l-2,2-dimethylcyclopropanecarboxylic acid is obtained by purifying the filtrate part, this result being opposite to the foregoing one obtained with d-α-phenyl-β-(p-tolyl)ethylamine.

In the optical purification of the present invention, as described above, the optical rotatory power of an optically active 2,2-dimethylcyclopropanecarboxylic acid obtained from the crystal part is not always dependent upon the optical rotatory power of the optically active diphenylethylamine used, but varies with the kind of the diphenylethylamine.

Further, when 2,2-dimethylcyclopropanecarboxylic acid contains either one of its d-form or l-form in excess, particularly in an excess of not less than 15%, preferably not less than 30%, an optically active 2,2-dimethylcyclopropanecarboxylic acid alone contained in optical excess is obtained as a diastereomer salt independently of the kind and optical rotatory power of the optically active diphenylethylamine used.

For example, when the 2,2-dimethylcyclopropanecarboxylic acid is a d-rich one, the crystals deposited as a diastereomer salt is always the salt of 3-2,2-dimethylcyclopropanecarboxylic acid independently of the kind and optical rotatory power of the optically active diphenylethylamine of the formula (I). As described above, when either one of the d-form or l-form is present in excess, its optical purification is achieved in a particularly efficient manner independently of the kind and optical rotatory power of the optically active diphenylethylamine used. Such a result does not appear to have been previously obtained.

Next, the present invention will be illustrated in more detail with reference to the following Examples.

Example 1

Thirty grams of 2,2-dimethylcyclopropanecarboxylic acid, 51.9 g of d-α-phenyl-β-phenylethylamine, 500 ml of isopropyl alcohol and 500 ml of water were fed to a flask, followed by refluxing for 30 minutes. Thereafter, 180 mg of the crystals of the diastereomer salt of d-2,2-dimethylcyclopropanecarboxylic acid with d-α-phenyl-β-phenylethylamine, as separately prepared, was added at 40°C as seed crystals. The contents of the flask were then slowly cooled to 20°C, and the deposited crystals were filtered off.

The crystals obtained were recrystallized from a mixed solvent of 250 ml of isopropyl alcohol and 250 ml of water to obtain 28.9 g of crystals.

To 28.9 g of these crystals were added 100 ml of ethyl ether and 25 ml of water, and to the resulting mixture was added 35.6 g of 10% aqueous hydrochloric acid to decompose the salt, whereby the d-2,2-dimethylcyclopropanecarboxylic acid released was extracted into the organic layer. The organic layer was washed with water, and after removing the ether by evaporation, the residual liquor was further distilled to obtain 10.3 g of d-2,2-dimethylcyclopropanecarboxylic acid (yield, 34.3%).

| | |
|---|---|
| $[\alpha]_D^{20}$ +147.1° (c=1, chloroform) | |
| Enantiometric excess of the d-form | 99.5% |
| Content | 99.2% |

The excess amount was obtained by esterifying the acid with l-menthol and measuring the diastereomer obtained by gas chromatography.

Example 2

Thirty grams of 2,2-dimethylcyclopropanecarboxylic acid, 55.5 g of d-α-phenyl-β-(p-tolyl)ethylamine, 240 ml of methanol and 60 ml of water were fed to a flask, followed by refluxing for 30 minutes. Thereafter, 180 mg of the crystals of the diastereomer salt of l-2,2-dimethylcyclopropanecarboxylic acid with d-α-phenyl-β-(p-tolyl)ethylamine, as separately prepared, was added at 35° to 40°C as seed crystals. The contents of the flask were then slowly cooled to 20°C, and the deposited crystals were filtered off.

The crystals obtained were recrystallized from a mixed solvent of 250 ml of methanol and 250 ml of water to obtain 28.5 g of crystals.

To 28.5 g of these crystals were added 100 ml of ethyl ether and 25 ml of water, and to the resulting mixture was added 33.6 g of 10% aqueous hydrochloric acid to decompose the salt, whereby the l-2,2-dimethylcyclopropanecarboxylic acid released was extracted into the organic layer.

The organic layer was washed with water, and after removing the ether by evaporation, the residual liquor was further distilled to obtain 9.79 g of l-2,2-dimethylcyclopropanecarboxylic acid (yield, 32.6%).

| | |
|---|---|
| $[\alpha]_D^{20}$ −146.7° (c=1, chloroform) | |
| Enantiometric excess of the l-form | 99.4% |
| Content | 99.3% |

Example 3

Thirty grams of 2,2-dimethylcyclopropanecarboxylic acid, 55.5 g of l-α-(p-tolyl)-β-phenylethylamine, 500 ml of isopropyl alcohol and 500 ml of water were fed to a flask, followed by refluxing for 30 minutes. Thereafter, 180 mg of the crystals of the diastereomer salt of d-2,2-dimethylcyclopropanecarboxylic acid with l-α-(p-tolyl)-β-phenylethylamine, as separately prepared, was added at 40°C as seed crystals. The contents of the flask were then slowly cooled to 20°C, and the deposited crystals were filtered off.

The crystals obtained were recrystallized from a mixed solvent of 250 ml of isopropyl alcohol and 250 ml of water to obtain 28.2 g of crystals.

To 28.2 g of these crystals were added 100 ml of ethyl ether and 25 ml of water, and to the resulting mixture was added 33.2 g of 10% aqueous hydrochloric acid to decompose the salt, whereby the d-2,2-dimethylcyclopropanecarboxylic acid released was extracted into the organic layer.

The organic layer was washed with water, and after removing the ether by evaporation, the residual liquor was further distilled to obtain 9.70 g of d-2,2-dimethylcyclopropanecarboxylic acid (yield, 32.3%).

| | |
|---|---|
| $[\alpha]_D^{20}$ +146.3° (c=1, chloroform) | |
| Enantiometric excess of the d-form | 99.2% |
| Content | 99.1% |

Example 4

Thirty grams of 2,2-dimethylcyclopropanecarboxylic acid, 55.5 g of l-α-phenyl-β-(p-tolyl)ethylamine, 240 ml of methanol and 60 ml of water were fed to a flask, followed by refluxing for 30 minutes. Thereafter, 180 mg of the crystals of the diastereomer salt of d-2,2-dimethylcyclopropanecarboxylic acid with l-α-phenyl-β-(p-tolyl)ethylamine, as separately prepared, was added at 40°C as seed crystals. The contents of the flask were then slowly cooled to 20°C, and the deposited crystals were filtered off.

The crystals obtained were recrystallized from a mixed solvent of 250 ml of methanol and 250 ml of water to obtain 28.6 g of crystals.

To 28.6 g of these crystals were added 100 ml of ethyl ether and 25 ml of water, and to the resulting mixture was added 33.7 g of 10% aqueous hydrochloric acid to decompose the salt, whereby the d-2,2-dimethylcyclopropanecarboxylic acid released was extracted into the organic layer.

The organic layer was washed with water, and after removing the ether by evaporation, the residual liquor was further distilled to obtain 9.83 g of d-2,2-dimethylcyclopropanecarboxylic acid (yield, 32.8%).

| | |
|---|---|
| $[\alpha]_D^{20}$ +146.4° (c=1, chloroform) | |
| Enantiometric excess of the d-form | 99.3% |
| Content | 99.3% |

Example 5

Thirty grams of 2,2-dimethylcyclopropanecarboxylic acid, 51.9 g of l-α-phenyl-β-phenylethylamine and 2 liters of benzene were fed to a flask, followed by refluxing for 30 minutes. Thereafter, 180 mg of the crystals of the diastereomer salt of l-2,2-dimethylcyclopropanecarboxylic acid with l-α-phenyl-β-phenylethylamine, as separately prepared, was added at 50° to 45°C as seed crystals. The contents of the flask were then slowly cooled to 20°C, and the deposited crystals were filtered off. The crystals obtained were recrystallized from a mixed solvent of 250 ml of isopropyl alcohol and 250 ml of water to obtain 28.6 g of crystals.

To 28.6 g of these crystals were added 100 ml of ethyl ether and 25 ml of water, and to the resulting mixture was added 35.2 g of 10% aqueous hydrochloric acid to decompose the salt, whereby the l-2,2-dimethylcyclopropanecarboxylic acid released was extracted into the organic layer. The organic layer was washed with water, and after removing the ether by evaporation, the residual liquor was further distilled to obtain 10.2 g of l-2,2-dimethylcyclopropanecarboxylic acid (yield, 34.0%).

| | |
|---|---|
| $[\alpha]_D^{20}$ −147.2° (c=1, chloroform) | |
| Enantiometric excess of the l-form | 99.4% |
| Content | 99.3% |

Example 6

After removing the deposited crystals by filtration in Example 2, the solvent was removed from the filtrate by evaporation by concentration under reduced pressure.

The solid obtained, 250 ml of methanol and 250 ml of water were fed to a flask, followed by refluxing for 30 minutes. Thereafter, 180 mg of the crystals of the diastereomer salt of l-2,2-dimethylcyclopropanecarboxylic acid with d-α-phenyl-β-(p-tolyl)ethylamine, as separately prepared, was added at 45° to 50°C as seed crystals. The contents of the flask were then slowly cooled to 20°C, and the deposited crystals were filtered off.

To 27.9 g of the crystals obtained were added 100 ml of ethyl ether and 25 ml of water, and to the resulting mixture was added 32.9 g of 10% aqueous hydrochloric acid to decompose the salt, whereby the d-2,2-dimethylcyclopropanecarboxylic acid released was extracted into the organic layer. The organic layer was washed with water, and after removing the ether by evaporation, the residual liquor was further distilled to obtain 9.59 g of d-2,2-dimethylcyclopropanecarboxylic acid (yield, 32.0%).

| | |
|---|---|
| $[\alpha]_D^{20}$ +146.5° (c=1, chloroform) | |
| Enantiometric excess of the d-form | 99.2% |
| Content | 99.3% |

Example 7

After removing the deposited crystals by filtration in Example 1, the solvent was removed from the filtrate by evaporation by concentration under reduced pressure.

The solid obtained, 250 ml of isopropyl alcohol and 250 ml of water were fed to a flask, followed by refluxing for 30 minutes. Thereafter, 180 mg of the crystals of the diastereomer salt of l-2,2-dimethylcyclopropanecarboxylic acid with d-α-phenyl-β-phenylethylamine, as separately prepared, was added at 45° to 50°C as seed crystals. The contents of the flask were then slowly cooled to 20°C, and the deposited crystals were filtered off.

To 27.5 g of the crystals obtained were added 100 ml of ethyl ether and 25 ml of water, and to the resulting mixture was added 33.8 g of 10% aqueous hydrochloric acid to decompose the salt, whereby the l-2,2-dimethylcyclopropanecarboxylic acid released was extracted into the organic layer. The organic layer was washed with water, and after removing the ether by evaporation, the residual liquor was further distilled to obtain 9.86 g of l-2,2-dimethylcyclopropanecarboxylic acid (yield, 32.9%).

| | |
|---|---|
| $[\alpha]_D^{20}$ −146.8° (c=1, chloroform) | |
| Enantiometric excess of the l-form | 99.3% |
| Content | 99.1% |

Example 8

After removing the deposited crystals by filtration in Example 5, the solvent was removed from the filtrate by evaporation.

The solid obtained, 300 ml of isopropyl alcohol and 200 ml of water were fed to a flask, followed by refluxing for 30 minutes. Thereafter, the reaction solution was allowed to cool to 15°C, and the deposited crystals were filtered off. To 24.8 g of the crystals obtained were added 100 ml of ether and 25 ml of water, and to the resulting mixture was added 30.4 g of 10% aqueous hydrochloric acid to decompose the salt, whereby the d-2,2-dimethylcyclopropanecarboxylic acid released was extracted into the organic layer. The organic layer was washed with water, and after removing the ether by evaporation, the residual liquor was further distilled to obtain 8.87 g of d-2,2-dimethylcyclopropanecarboxylic acid (yield, 29.6%).

| | |
|---|---|
| $[\alpha]_D^{20}$ +145.2° (c=1, chloroform) | |
| Enantiometric excess of the d-form | 98.6% |
| Content | 99.0% |

Example 9

Thirty grams of 2,2-dimethylcyclopropanecarboxylic acid (excess amount of the d-form, 58.4%), 55.5 g of d-α-phenyl-β-(p-tolyl)ethylamine and 2 liters of benzene were fed to a flask, followed by refluxing for 1 hour. Thereafter, 180 mg of the crystals of the diastereomer salt of d-2,2-dimethylcyclopropanecarboxylic acid with d-α-phenyl-β-(p-tolyl)ethylamine, as separately prepared, was added at 30°C to 40°C as seed crystals. The contents of the flask were then slowly cooled to 10°C, and the deposited crystals were filtered off.

The crystals obtained were further recrystallized from 18 times their weight, expressed in parts by weight, of benzene to obtain 44.1 g of crystals.

Thereafter, to a mixed liquor comprising 43.0 g of the salt obtained here, 200 ml of ethyl ether and 50 ml of water was added 56 g of 10% aqueous hydrochloric acid to decompose the salt, whereby the d-2,2-dimethylcyclopropanecarboxylic acid released was extracted into the organic layer. The organic layer was washed with water, and after removing the ether by evaporation, the residual liquor was distilled to obtain 14.6 g of d-2,2-dimethylcyclopropanecarboxylic acid (yield, 50% based on the fed carboxylic acid).

| | |
|---|---|
| $[\alpha]_D^{20}$ 145.1° (c=1, chloroform) | |
| Enantiometric excess of the d-form | 98.4% |

Example 10

Fifteen grams of 2,2-dimethylcyclopropanecarboxylic acid (excess amount of the d-form, 73.9%), 240 ml of isopropyl alcohol, 320 ml of water and 27.8 g of d-α-phenyl-β-(p-tolyl)ethylamine were added to a flask, followed by refluxing for 30 minutes. Thereafter, 90 mg of the same seed crystals as used in Example 9 were added at 45°C to 50°C, the contents of the flask were slowly cooled to 15°C and the deposited crystals were filtered off. Thus, 30.5 g of a salt was obtained.

Thereafter, 25 g of the salt obtained here, 120 ml of ethyl ether and 30 ml of water were fed to a flask, and 32 g of 10% aqueous hydrochloric acid was added to decompose the salt. The subsequent after-treatment, purification and distillation of the residue obtained were carried out as described in Example 9.

Amount obtained, 8.63 g (yield, 70.2% based on the fed carboxylic acid)

| | |
|---|---|
| $[\alpha]_D^{20}$ 146.4° (c=1, chloroform) | |
| Enantiometric excess of the d-form | 99.8% |
| Content | 98.9% |

Example 11

11.4 Grams of 2,2-dimethylcyclopropanecarboxylic acid (excess amount of the d-form, 81.5%), 21.1 g of d-α-phenyl-β-(p-tolyl)ethylamine and 500 ml of toluene were fed to a flask, followed by refluxing for 1 hour. Thereafter, 60 mg of the same seed crystals as used in Example 10 was added at 55° to 60°C, the contents of the flask were slowly cooled to 20°C and the deposited crystals were filtered off.

The crystals obtained were further recrystallized from 15 times their weight, expressed in parts by weight, of toluene to obtain 20.5 g of crystals.

Thereafter, the crystals obtained here, 50 ml of toluene and 30 ml of water were fed to a flask, and 27 g of 10% aqueous hydrochloric acid was added to the mixture to decompose the salt. The subsequent after-treatment and purification were carried out as described in Example 9.

Amount obtained, 7.81 g (yield, 68.5%)

| | |
|---|---|
| Enantiometric excess of the d-form | 98.3% |

## Example 12

A reaction was carried out in the same manner as in Example 11 except that 500 ml of toluene was replaced by 200 ml of methanol plus 200 ml of water, and the resulting mixture was slowly cooled to 20°C. The amount of the deposited crystals was 19.7 g.

Thereafter, 19 g of the salt obtained here, 50 ml of toluene and 30 ml of water were fed to a flask, and 26 g of 10% aqueous hydrochloric acid was added to the mixture to decompose the salt. The subsequent after-treatment and purification were carried out as described in Example 10.

Amount obtained 6.63 g (yield 60.3%)

| | |
|---|---|
| Enantiometric excess of the d-form | 97.8% |

## Example 13

22.8 Grams of 2,2-dimethylcyclopropanecarboxylic acid (excess amount of the d-form, 75.1%), 38.0 g of d-α-phenyl-β-(p-tolyl)ethylamine, 400 ml of water and 400 ml of isopropyl alcohol were added to a flask, followed by refluxing for 30 minutes. Thereafter, 140 mg of the same seed crystals as used in Example 9 was added at 45°C, the contents of the flask were slowly cooled to 15°C, and the deposited crystals were filtered off. Thus, 44.9 g of a salt was obtained.

Thereafter, 32.5 g of the salt obtained here, 150 ml of toluene and 50 ml of water were fed to a flask, and 24 g of 20% sodium hydroxide was added to the mixture to decompose the salt. The aqueous layer was separated, 46.8 g of 10% hydrochloric acid was added thereto, and the released carboxylic acid was extracted with 80 ml of ethyl ether. The subsequent after-treatment, purification and distillation of the residue obtained were carried out as described in Example 9. From the toluene layer was recovered d-α-phenyl-β-(p-tolyl)ethylamine.

Amount obtained 11.28 g (yield, 68.3% based on the fed carboxylic acid)

$[\alpha]_D^{20}$ 146.1° (c=1, chloroform)

| | |
|---|---|
| Enantiometric excess of the d-form | 99.7% |
| Content | 98.7% |

## Example 14

Thirty grams of 2,2-dimethylcyclopropanecarboxylic acid (excess amount of the d-form, 70%), 55.5 g of l-α-phenyl-β-(p-tolyl)ethylamine, 500 ml of isopropyl alcohol and 500 ml of water were fed to a flask, followed by refluxing for 30 minutes. Thereafter, 180 mg of the crystals of the diastereomer salt of d-2,2-dimethylcyclopropanecarboxylic acid with l-α-phenyl-β-(p-tolyl)ethylamine, as separately prepared, was added at 50° to 52°C as seed crystals. The contents of the flask were then slowly cooled to 20°C, and the separated crystals were filtered off.

To 55.2 g of the crystals obtained were added 200 ml of ethyl ether and 50 ml of water, and to the resulting mixture was added 65 g of 10% aqueous hydrochloric acid to decompose the salt, whereby the d-2,2-dimethylcyclopropanecarboxylic acid released was extracted into the organic layer.

The organic layer was washed with water, and after removing the ether by evaporation, the residual liquor was further distilled to obtain 19.6 g of d-2,2-dimethylcyclopropanecarboxylic acid (yield, 65.3%).

$[\alpha]_D^{20}$ +147.0° (c=1, chloroform)

| | |
|---|---|
| Enantiometric excess of the d-form | 99.4% |
| Content | 99.3% |

## Example 15

Thirty grams of 2,2-dimethylcyclopropanecarboxylic acid (excess amount of the d-form, 50%), 55.5 g of l-α-phenyl-β-(p-tolyl)ethylamine and 2 liters of benzene were fed to a flask, followed by refluxing for 1 hour.

Thereafter, 180 mg of the same seed crystals as used in Example 14 was added, the contents of the flask were slowly cooled to 10°C, and the separated crystals were filtered off.

The crystals obtained were recrystallized from 20 times their weight, expressed in parts by weight, of benzene to obtain 39.2 g of crystals.

To 39.2 g of these crystals were added 150 ml of ethyl ether and 40 ml of water, and to the resulting mixture was added 46 g of 10% aqueous hydrochloric acid to decompose the salt.

In the same manner as that described in Example 14, the subsequent after-treatment and purification were applied to obtain 13.7 g of d-2,2-dimethylcyclopropanecarboxylic acid (yield, 45.7%).

$[\alpha]_D^{20}$ +146.2° (c=1, chloroform)

| | |
|---|---|
| Enantiometric excess of the d-form | 99.1% |
| Content | 99.4% |

Example 16

Thirty grams of 2,2-dimethylcyclopropanecarboxylic acid (enantiometric excess of the d-form, 80%), 51.9 g of l-α-phenyl-β-phenylethylamine, 500 ml of methyl alcohol and 500 ml of water were fed to a flask, followed by refluxing for 30 minutes. Thereafter, 180 mg of the crystals of the diastereomer salt of d-2,2-dimethylcyclopropanecarboxylic acid with l-α-phenyl-β-phenylethylamine, as separately prepared, was added at 45° to 50°C as seed crystals. The contents of the flask were then slowly cooled to 20°C, and the deposited crystals were filtered off.

To 54.1 g of the crystals obtained were added 200 ml of ethyl ether and 50 ml of water, and to the resulting mixture was added 66.5 g of 10% aqueous hydrochloric acid to decompose the salt. In the same manner as that described in Example 14, the subsequent after-treatment and purification were applied to obtain 19.3 g of d-2,2-dimethylcyclopropanecarboxylic acid (yield, 64.3%).

$[\alpha]_D^{20}$ +146.8° (c=1, chloroform)

| | |
|---|---|
| Enantiometric excess of the d-form | 99.5% |
| Content | 99.2% |

Example 17

Thirty grams of 2,2-dimethylcyclopropanecarboxylic acid (enantiometric excess of the l-form, 70%), 55.5 g of l-α-phenyl-β-(p-tolyl)ethylamine, 500 ml of methyl alcohol and 500 ml of water were fed to a flask, followed by refluxing for 30 minutes. Thereafter, 180 mg of the crystals of the diastereomer salt of l-2,2-dimethylcyclopropanecarboxylic acid with l-α-phenyl-β-(p-tolyl)ethylamine, as separately prepared, was added at 50° to 52°C as seed crystals. The contents of the flask were then slowly cooled to 20°C, and the deposited crystals were filtered off.

To 54.3 g of the crystals obtained were added 200 ml of ethyl ether and 50 ml of water, and to the resulting mixture was added 64 g of 10% aqueous hydrochloric acid to decompose the salt, whereby the l-2,2-dimethylcyclopropanecarboxylic acid released was extracted into the organic layer. The organic layer was washed with water, and after removing the ether by evaporation, the residual liquor was further distilled to obtain 18.5 g of l-2,2-dimethylcyclopropanecarboxylic acid (yield, 61.7%).

$[\alpha]_D^{20}$ −146.3° (c=1, chloroform)

| | |
|---|---|
| Enantiometric excess of the l-form | 99.5% |
| Content | 98.7% |

Example 18

Thirty grams of 2,2-dimethylcyclopropanecarboxylic acid (enantiometric excess of the l-form, 50%), 55.5 g of d-α-phenyl-β-(p-tolyl)ethylamine, 500 ml of methyl alcohol and 500 ml of water were fed to a flask, followed by refluxing for 30 minutes. Thereafter, 180 mg of the crystals of the salt of l-2,2-dimethylcyclopropanecarboxylic acid with d-α-phenyl-β-(p-tolyl)ethylamine, as separately prepared, was added at 48° to 50°C as seed crystals. The contents of the flask were then slowly cooled to 20°C, and the deposited crystals were filtered off.

To 38.5 g of the crystals obtained were added 150 ml of ethyl ether and 40 ml of water, and to the resulting mixture was added 45.3 g of 10% aqueous hydrochloric acid to decompose the salt. The subsequent aftertreatment and purification were applied as described in Example 17 to obtain 13.2 g of l-2,2-dimethylcyclopropanecarboxylic acid (yield, 44%).

$[\alpha]_D^{20}$ −147.0° (c=1, chloroform)

| | |
|---|---|
| Enantiometric excess of the l-form | 99.5% |
| Content | 99.2% |

Example 19

Thirty grams of 2,2-dimethylcyclopropanecarboxylic acid (enantiometric excess of the l-form, 40%), 51.9 g of l-α-phenyl-β-phenylethylamine and 2 liters of benzene were fed to a flask, followed by refluxing for 1 hour. Thereafter, 180 mg of the crystals of the salt of l-2,2-dimethylcyclopropanecarboxylic acid with l-α-phenyl-β-phenylethylamine, as separately prepared, was added at 30° to 40°C as seed crystals. The contents of the flask were then slowly cooled to 10°C, and the deposited crystals were filtered off.

The crystals obtained were recrystallized from 20 times their weight, expressed in parts by weight, of benzene to obtain 33.2 g of crystals.

To 33.2 g of the crystals obtained here were added 150 ml of ethyl ether and 40 ml of water, and to the resulting mixture was added 41 g of 10% aqueous hydrochloric acid to decompose the salt. The subsequent after-treatment and purification were applied as described in Example 17 to obtain 11.9 g of l-2,2-dimethylcyclopropanecarboxylic acid (yield, 39.7%).

$[\alpha]_D^{20}$ −146.8° (c=1, chloroform)

| | |
|---|---|
| Enantiometric excess of the l-form | 99.7% |
| Content | 99.0% |

Example 20

Thirty grams of 2,2-dimethylcyclopropanecarboxylic acid (enantiometric excess of the l-form, 80%), 51.9 g of d-α-phenyl-β-phenylethylamine, 500 ml of methyl alcohol and 500 ml of water were fed to a flask, followed by refluxing for 30 minutes. Thereafter, 180 mg of the crystals of the salt of l-2,2-dimethylcyclopropanecarboxylic acid with d-α-phenyl-β-phenylethylamine, as separately prepared, was added at 45° to 50°C as seed crystals. The contents of the flask were then slowly cooled to 20°C, and the deposited crystals were filtered off.

To 54.9 g of the crystals obtained were added 200 ml of ethyl ether and 50 ml of water, and to the resulting mixture was added 67.5 g of 10% aqueous hydrochloric acid to decompose the salt. The subsequent after-treatment and purification were applied as described in Example 17 to obtain 20 g of l-2,2-dimethylcyclopropanecarboxylic acid (yield, 66.7%).

| | |
|---|---|
| $[\alpha]_D^{20}$ −147.2° (c=1, chloroform) | |
| Enantiometric excess of the l-form | 99.8% |
| Content | 99.3% |

Example 21

After removing the obtained crystals by filtration in Example 11, the solvent was removed from the filtrate by evaporation by concentration under reduced pressure.

The solid obtained, 75 ml of methanol and 50 ml of water were fed to a flask, followed by refluxing for 30 minutes. Thereafter, 30 mg of the crystals of the diastereomer salt of d-2,2-dimethylcyclopropanecarboxylic acid with d-α-phenyl-β-(p-tolyl)ethylamine, as separately prepared, was added at 45° to 50°C as seed crystals. The contents of the flask were then slowly cooled to 20°C, and the deposited crystals were filtered off.

To 6.30 g of the crystals obtained were added 40 ml of ethyl ether and 15 ml of water, and to the resulting mixture was added 7.5 g of 10% aqueous hydrochloric acid to decompose the salt, whereby the d-2,2-dimethylcyclopropanecarboxylic acid released was extracted into the organic layer. The subsequent after-treatment and purification were applied as described in Example 1 to obtain 2.14 g of d-2,2-dimethylcyclopropanecarboxylic acid (yield, 18.8% based on the fed 2,2-dimethylcyclopropanecarboxylic acid).

| | |
|---|---|
| $[\alpha]_D^{20}$ +147.4° (c=1, chloroform) | |
| Enantiometric excess of the d-form | 99.5% |
| Content | 99.3% |

Comparative example 1

To a four-necked flask equipped with a stirrer and a thermometer were fed 17.1 g of 2,2-dimethylcyclopropanecarboxylic acid and 1.5 liters of benzene, and then a solution of 18.2 g of d(+)-α-methylbenzylamine in 750 ml of benzene was added thereto at room temperature.

After 20 hours, the crystals were collected by filtration and recrystallized four times from benzene to obtain 5.3 g of crystals. Thereafter, these crystals were decomposed with 70 ml of N-aqueous hydrochloric acid, followed by extraction with 150 ml of ether. The ether layer was washed with water and dried over magnesium sulfate. After removing magnesium sulfate by filtration, the ether layer obtained was concentrated to obtain 1.82 g of (−)-2,2-dimethylcyclopropanecarboxylic acid (yield, 10.6%).

Optical rotation $[\alpha]_D^{20}$ −70.8° (c=1.0, chloroform)
Optical purity 47.7%

Comparative example 3

Reaction was carried out using the same raw materials, amounts used and operation as used in Comparative example 1 except that d(+)-α-methylbenzylamine was replaced by l(−)-α-methylbenzylamine. Thus, 5.9 g of crystals were obtained.

These crystals were decomposed with 80 ml of N-aqueous hydrochloric acid, followed by extraction with 150 ml of ether. In the same manner as in Comparative example 1, the subsequent after-treatment and purification were applied to obtain 1.91 g of (+)-2,2-dimethylcyclopropanecarboxylic acid (yield, 11.2%).

Optical rotation $[\alpha]_D^{26}$ +66.0° (c=1.0, chloroform)
Optical purity 44.9%

Comparative example 3

To the same apparatus as used in Comparative example 1 were added 23.1 g of 2,2-dimethylcyclopropanecarboxylic acid and 33 ml of water, and the pH of the resulting mixture was adjusted to 8.0 with about 10 ml of 50% aqueous sodium hydroxide solution. Thereafter, a solution of 38.4 g of quinine in a mixture of 60 ml of methanol and 30 ml of water, the pH of the water being previously adjusted to 7.1 by adding about 8 ml of conc. hydrochloric acid (this solution is in fact a quinine hydrochloride solution), was added to the foregoing reaction mixture which was then turned into solution by heating. On cooling the

solution to 20°C, crystals deposited. This solution was allowed to stand for two days at room temperature. Thereafter, the crystals were collected by filtration, washed with two 10-ml portions of water and then with two 10-ml portions of 50% aqueous ethanol to obtain 41.3 g of crude crystals. These crude crystals were recrystallized from acetone to obtain 24.3 g of crystals. This quinine salt was decomposed with 80 ml of 2% aqueous alkali solution and 100 ml of chloroform, and the resulting aqueous layer was acidified with 20% aqueous hydrochloric acid and then extracted with 100 ml of ether. The organic layer was washed with water, dried over magnesium sulfate and concentrated to obtain 4.1 g of (+)-2,2-dimethylcyclopropanecarboxylic acid (yield, 17.7%).

Optical rotation +143.0° (c=1, chloroform)
Optical purity 97.2%

**Claims**

1. A metod of recovering from 2,2-dimethylcyclopropanecarboxylic acid, an optically active form thereof, which method comprises reacting an optically active diphenylethylamine represented by the formula (I),

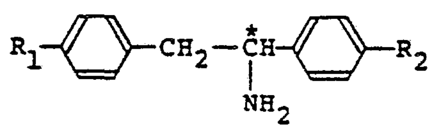

(I)

wherein $R_1$ and $R_2$ are a hydrogen atom or a lower alkyl group, with the 2,2-dimethylcyclopropanecarboxylic acid in a solvent to form a diastereomer salt with the said amine, allowing the diastereomer salt to deposit, separating the said diastereomer salt from the solvent to thereby separate the optically active forms of the said acid from one another and recovering the desired said optically active form from one of the said salt and the said solvent.

2. A method according to claim 1, wherein the desired said optically active form is recovered from the said deposited diastereomer salt by decomposition thereof.

3. A method according to claim 1 or claim 2, wherein the said 2,2-dimethylcyclopropanecarboxylic acid from which the said optically active form is to be recovered contains the d-form in excess, and the said optically active 2,2-dimethylcyclopropanecarboxylic acid obtained is d-2,2-dimethylcyclopropanecarboxylic acid.

4. A method according to claim 1 or claim 2, wherein the said 2,2-dimethylcyclopropanecarboxylic acid from which the said optically active form is to be recovered contains the l-form in excess, and the said optically active 2,2-dimethylcyclopropanecarboxylic acid obtained is l-2,2-dimethylcyclopropanecarboxylic acid.

5. A method according to claim 3 or claim 4, wherein the excess of an optically active form present in the said 2,2-dimethylcyclopropanecarboxylic acid from which the said optically active form is to be recovered is not less than 30%.

6. A method according to any one of the preceding claims, wherein the said optically active diphenylethylamine is d- or l-α-phenyl-β-(p-tolyl)ethylamine, d- or l-α-(p-tolyl)-β-phenylethylamine or d- or l-α-phenyl-β-phenylethylamine.

7. A method according to any one of the preceding claims, wherein the molar amount of the optically active diphenylethylamine used is from 0.3 to 1.2 times the molar amount of the said 2,2-dimethylcyclopropanecarboxylic acid from which the optically active form thereof is to be recovered.

**Patentansprüche**

1. Verfahren zur Gewinnung von 2,2-Dimethylcyclopropancarbonsäure in optisch aktiver Form, dadurch gekennzeichnet, daß man ein optisch aktives Diphenylethylamin der Formel

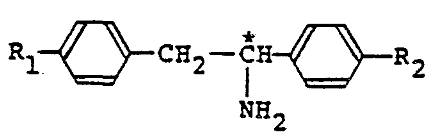

(I)

worin $R_1$ und $R_2$ für ein Wasserstoffatom oder eine kurzkettige Alkylgruppe stehen, in einem Lösungsmittel mit der 2,2-Dimethylcyclopropancarbonsäure zur Bildung ein Diastereoisomerensalzes mit dem Amin umsetzt, das Diastereoisomerensalz sich absetzen läßt und das Diastereoisomerensalz von Lösungsmittel abtrennt, um dadurch die optisch aktiven Formen der Säure voneinander zu trennen und die speziell gewünschte optisch aktive Form von einer des Salzes und des Lösungsmittel zurückzugewinnen.

2. Verfahren nach Anspruch 1, dadurch gekennzeichnet, daß man die speziell gewünschte optisch aktive Form aus dem abgeschiedenen Diastereoisomerensalz durch Zersetzung desselben zurückgewinnt.

3. Verfahren nach Anspruch 1 oder 2, dadurch gekennzeichnet, daß die 2,2-Dimethylcyclopropansäure, aus der die optisch aktive Form dargestellt werden soll, die d-Form im Überschuß enthält und daß die erhaltene optisch aktive 2,2-Dimethylcyclopropancarbonsäure aus d-2,2-Dimethylcyclopropancarbonsäure besteht.

4. Verfahren nach Anspruch 1 oder 2, dadurch gekennzeichnet, daß die 2,2-Dimethylcyclopropancarbonsäure, aus der die optisch aktive Form dargestellt werden soll, die l-Form im Überschuß enthält und die erhaltene optisch aktive 2,2-Dimethylcyclopropancarbonsäure aus l-2,2-Dimethylcyclopropancarbonsäure besteht.

5. Verfahren nach Anspruch 3 oder 4, dadurch gekennzeichnet, daß der Überschuß an einer optisch aktiven Form in der 2,2-Dimethylcyclopropancarbonsäure, aus der diese optisch aktive Form gewonnen werden soll, nicht weniger als 30% beträgt.

6. Verfahren nach einem der vorhergehenden Ansprüche, dadurch gekennzeichnet, daß das optisch aktive Diphenylethylamin d- oder l-α-Phenyl-β-(p-tolyl)-ethylamin, d- oder l-α-(p-Tolyl)-β-phenylethylamin oder d- oder l-α-Phenyl-β-phenylethylamin ist.

7. Verfahren nach einem der vorhergehenden Ansprüche dadurch gekennzeichnet, daß die molare Menge des verwendeten optisch aktiven Diphenylethylamins das 0,3- bis 1,2-fache der molaren Menge der 2,2-Dimethylcyclopropancarbonsäure, aus der ihre optisch aktive Form gewonnen werden soll, beträgt.

**Revendications**

1. Procédé d'obtention, à partir de l'acide 2,2-diméthylcyclopropanecarboxylique, d'une de ses formes optiquement actives, procédé qui consiste à faire réagir une diphényléthylamine optiquement active de formule (I)

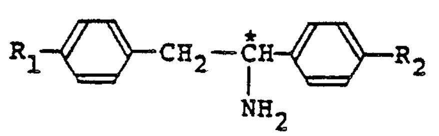

(I)

dans laquelle $R_1$ et $R_2$ sont des atomes d'hydrogène ou des groupements alkyle inférieur, avec l'acide 2,2-diméthylcyclopropanecarboxylique dans un solvant pour former un sel diastéréoisomère avec ladite amine, à laisser le sel diastéréoisomère se déposer, à séparer ledit sel diastéréoisomère du solvant de façon à séparer les formes optiquement actives dudit acide l'une de l'autre et à obtenir ladite forme optiquement active désirée à partir dudit sel ou dudit solvant.

2. Procédé selon la revendication 1, dans lequel ladite forme optiquement active désirée est obtenue par décomposition dudit sel diastéréoisomère déposé.

3. Procédé selon la revendication 1 ou 2, dans lequel ledit acide 2,2-diméthylcyclopropanecarboxylique à partir duquel on cherche à obtenir ladite forme optiquement active contient un excès de la forme d, et ledit acide 2,2-diméthylcyclopropanecarboxylique optiquement actif obtenu est l'acide d-2,2-diméthylcyclopropanecarboxylique.

4. Procédé selon la revendication 1 ou 2, dans lequel ledit acide 2,2-diméthylcyclopropanecarboxylique à partir duquel on cherche à obtenir ladite forme optiquement active contient un excès de la forme l, et ledit acide 2,2-diméthylcyclopropanecarboxylique optiquement actif obtenu est l'acide l-2,2-diméthylcyclopropanecarboxylique.

5. Procédé selon la revendication 3 ou 4, dans lequel l'excès de la forme optiquement active présente dans ledit acide 2,2-diméthylcyclopropanecarboxylique à partir duquel on cherche à obtenir ladite forme optiquement active n'est pas inférieur à 30%.

6. Procédé selon l'une quelconque des revendications 1 à 5, dans lequel ladite diphényléthylamine optiquement active est la d- ou l-α-phényl-β-(p-tolyl) éthylamine, la d- ou l-α-(p-tolyl)-β-phényléthylamine ou la d- ou l-α-phényl-β-phényléthylamine.

7. Procédé selon l'une quelconque des revendications 1 à 6, dans lequel la quantité molaire de diphényléthylamine optiquement active utilisée est de 0,3 à 1,2 fois la quantité molaire dudit acide 2,2-diméthylcyclopropanecarboxylique à partir duquel on cherche à obtenir ladite forme optiquement active.